# EUROPEAN PATENT SPECIFICATION

(11) **EP 0 462 630 B1**
(45) Date of publication and mention of the grant of the patent: **01.05.1996**
(21) Application number: 91113528.3
(22) Date of filing: 24.12.1987
(51) Int. Cl.: A01H 4/00

(54) **Method of multiplicating plant seedlings**
Verfahren zum Vermehren von Pflanzensetzlingen
Procédé de multiplication de jeunes plantes

(30) Priority: 26.12.1986 JP 308539/86; 26.12.1986 JP 308540/86; 26.06.1987 JP 157809/87
(43) Date of publication of application: 27.12.1991
(62) Divisional of application: 87311472.2
(73) Proprietor: MITSUI PETROCHEMICAL INDUSTRIES, LTD., Tokyo 100 (JP)
(72) Inventor: Tanimoto, Shizufumi, Saga-shi, Saga-ken (JP); Takahashi, Shigeru, Otake-shi, Hiroshima 739-06 (JP)
(74) Representative: Cresswell, Thomas Anthony

(56) References cited:
- US-A- 4 548 901
- WORLD PATENTS INDEX LATEST Week 8742, Derwent Publications Ltd., London, GB; AN 87-295978 & JP-62 208 220 (MITSUI PETROCHEM IND) 12 September 1987

## Description

The present invention concerns a method of multiplicating seedlings in a great amount by effecting tissue culture on plants in accordance with a specific method.

Vegetables such as cabbages, tomatoes, cucumbers and rices have been utilized for foods. Horticultural plants such as tulips, bluebottle and rudbeckia have been favored as ornamental plants. These plants have been multiplicated so far by means of seeding or division of bulbs or tuber. However, such multiplication methods not only require a large area and much labour but also involve such problems as slow seeding growing rate or poor flower quality due to the spread of virus diseases. With the aim of overcoming these problems the technique of plant tissue culture has been used in recent years (for example see JP-A-55-14734). Multiplication by tissue culture has been attained by the differentiation of adventitious bud, adventitious embryo or bulb from cultured tissue pieces and cultured cells and it has been considered that the differentiation is controlled by the concentration ratio of cytokinin and auxin as plant hormone (for example, refer to Annals of Botany vol. 45, 321 - 327, 1980). However, there are many plant groups which do not undergo differentiation using plant hormones or the frequency of differentiation, if it occurs, is extremely low and there is a need for a more direct and effective method of inducing differentiation.

We have studied a method of more efficiently multiplicating seedlings of plants as compared with the usual case.

According to the invention there is provided a method of multiplicating seedlings of a plant which comprises treating tissue pieces or cultured cells of a plant belonging to Papaveraceae, Solanaceae except for Torenia, Umbelliferae, Rosaceae, Liliaceae except for Lilium, Compositae, Geraniaceae, Cucurbitaceae or Gramineae in a gas atmosphere containing 0 to less than 5% oxygen at a temperature from 15 to 30°C for 30 to 90 minutes and tissue culturing the treated tissue pieces or cultured cells in a medium not containing at least one calcium ionophore or cyclic AMP.

Specific examples of plants which can be used include those described in "Ground for the plants System Classification" edited by Yamagishi, published by Kokuryukan in 1974. More specifically, there can be mentioned plants belonging to Solanaceae such as egg plant, tomato, potato, sweet potato and basil, plants belonging to Papaveraceae such as poppy, rape, cabbage, radish and Chinese cabbage, plants belonging to Umbelliferae such as carrot, Japanese parsley and parsley, plants belonging to Rosaceae such as rose, strawberry, soybean and cherry, plants belonging to Liliaceae except for Lilium such as onions and tulips, plants belonging to Compositae such as chrysanthemum, bluebottle and sunflower, plants belonging to Geraniaceae such as pelargonium, geranium and flax, plants belonging to Cucurbitaceae such as cucumber and pumpkin and plants belonging to Gramineae such as rice and corn. Among these plants relevant to the present invention, preferred plants are egg plant, potato, carrot, cabbage, onion, soybean, radish, basil, bluebattle, rudbeckia, tulip, flax, cucumber and rice.

In the present invention, the tissue culture of plants can be conducted by using tissue pieces or cultured cells of the plant. The tissue pieces for culture can include, for example, tissue pieces of plants prepared by slicing cotyledon, hypocotyl, shoot apex, stalk, leaf, scale, root or other tissue. These tissue pieces are usually used after sterilization with sodium hypochlorite or ethanol. However, when using an aseptically cultured plant, a sterilized procedure is not required. Further, when multiplicating seedlings of plants with no disease or virus, tissues from near the apical point, that is tissue pieces of the plants obtained from tissue near the apical point can be used as the culture material. The cultured cells that can be used in the method of the present invention are not differentiated but are amorphous cells including callus tissue.

By the method of the present invention, differentiation of the tissue or the cultured cells of a plant into an adventitious embryo or bulb is significantly promoted.

The culture medium contains inorganic ingredients and a carbon source as essential ingredients, to which plant hormones and vitamins and, as required, amino acids can be added. The inorganic ingredients for the culture medium can include those inorganic salts including elements such as nitrogen, phosphor, potassium, sodium, calcium, magnesium, sulfur, iron, manganese, zinc, boron, molybdenum, chlorine, iodine and cobalts. Specifically, there can be mentioned those compounds such as potassium nitrate, sodium nitrate, ammonium nitrate, ammonium chloride, potassium chloride, calcium chloride, potassium monohydrogen phosphate, sodium dihydrogen phosphate, magnesium sulfate, magnesium chloride, sodium sulfate, ferrous sulfate, ferric sulfate, manganese sulfate, copper sulfate, sodium molybdate, molybdenum trioxide, potassium iodide, zinc sulfate, boric acid and cobalt chloride.

The carbon source for the culture medium can include, for example, carbon hydrate and derivatives thereof such as sucrose, organic acids such as fatty acid and primary alcohols such as ethanol.

Plant hormones for the culture medium can include, for example, auxins such as naphthalene acetic cid (NAA), indole acetic acid (IAA), p-chlorophenoxyacetic acid, 2,4-dichlorophenoxy acetic acid (2,4-D), indole acetic acid (IBA) and derivative thereof, and cytokinins such as benzyl adenine (BA), kinetin, zeatin, etc.

Vitamins for the culture medium can include, for example, biotine, thiamine (vitamin B1), pyridoxine (vitamin B6), pyridoxal, pyridoxamine, calcium pantotate, ascorbic acid (vitamin C), inositol, nicotinic acid, nicotinic amide and riboflavine (vitamin B2).

The amino acid for the culture medium can include, for example, glycine, alanine, glutamic acid, cystein, phenyl alanine and lysine.

The culture medium of the present invention is desirably used by usually incorporating from about 0.1 uM about 100 mM of the inorganic ingredient, from about 1 g/l to about 100 g/l of the carbon source, from about 0.01 mg/l to about 10 mg/l of the plant hormones, from about 0.1 mg/l to about 150 mg/l of the vitamin and from 0 to about 1000 mg/l of the amino acids.

The culture medium used for the tissue culture according to the present invention can include specifically those known culture media used for the tissue culture, for example, Murashige & Skoog culture medium ('62), Linsmaier & Skoog culture medium (RM-1965), White culture medium ('63), Gamborg B-5 culture medium, Mitsui M-9 culture medium, Nitch & Nitch culture medium, etc., being, preferably, incorporated as required with carbon sources and plant hormones as described above and, further, vitamins and amino acids as described above. The compositions of the known culture media mentioned above are described, for example, in "New Plant Tissue Culture" p386 - p391, written by Takeuchi, Nakajima, Furuya, and published from Asakura Shoten in 1979.

The culture medium used in the present invention is a liquid culture medium or solid culture medium usually containing from 0.5 to 1 % of agar.

The tissue pieces or cultured cells of plants as described above can be subjected to tissue culture using a liquid culture medium aerated with oxygen-containing gas in the same manner as described in Japanese published application number 61-285928, following treatment in accordance with the invention.

According to the method of the present invention, it is possible to obtain a larger amount of adventitious buds, adventitious embryos or bulblets (small bulbs), at high efficiency from tissue pieces or cultured cells of plants. Adventitious buds and adventitious embryos obtained by the method according to the invention can be rooted to provide a plant body, which can then be sliced into tissue pieces (a bulblet can also be sliced) and subjected to further tissue culture in accordance with the present invention to enable seedlings to be prepared in a great amount. Further, plants obtained by the present invention can be grown into intact plant bodies of stable nature by usual cultivation.

Plant bodies of high quality can be obtained in a large amount by the method of the invention at a better efficiency as compared with conventional methods.

The object of the present invention is attained by applying aerophobic treatment to the tissue pieces or cultured cells of each of plants belonging to Papaveraceae, Solanaceae except for Torenia, Umbelliferae, Rosaceae, Liliaceae except for Lilium, Compositae, Geraniaceae, Cucurbitaceae and Gramineae, and then applying tissue culture. That is, the method of multiplicating the seedlings of plants described later concerns a method of multiplicating the seedlings of plants by applying aerophobic treatment to tissue pieces or cultured cells of plants belongings to Papaveraceae, Solanaceae except for Torenia, Umbelliferae, Rosaceae, Liliaceae except for Lilium, Compositae, Geraniaceae, Cucurbitaceae and Gramineae followed by applying tissue culture.

In the method as described above, differentiation of adventitious buds, adventitious embryoes and bulbs from the tissue pieces or cultured cells of plants is improved. This method is based on a novel finding by the present inventors.

Details for the plants applicable with the above-mentioned method, as well as means for obtaining the tissue pieces or cultured cells from the above-mentioned plants are already described above.

The aerophobic treatment for the method described above can be conducted as described below. After collecting the tissue pieces of plants used for the tissue culture, specimens are placed in a gas atmosphere such as of nitrogen, argon or CO₂ not containing oxygen or containing oxygen usually of less than 5% such that the specimens are brought into contact with the gas usually at a temperature from 15 to 30°C for 30 to 90 minutes thereby applying aerophobic treatment. In the present invention, it is particularly desirable to apply the aerophobic treatment just after the collection of the tissue pieces. In the case of conducting the tissue culture using such tissue pieces, seedlings of the plants can preferably be multiplicated at high efficiency as compared with the case of using those applied with aerophobic treatment after the elapse of tissue from the collection.

As the culture media used for the tissue culture applied after the aerophobic treatment, those media described specifically later are used.

The medium that can be used in the method described above comprises inorganic ingredients and carbon source as the essential ingredient, and incorporated with plant hormones and vitamins and further with amino acids as required.

Examples of the inorganic ingredients, carbon sources, plant hormones, vitamins and amino acids in the culture medium, as well as the ratio of the ingredients in the culture medium, the kind of the basic culture medium and the preparation of the culture medium using them are the same as those already described for the present invention.

According to the method as described above, it is possible to obtain adventitious buds, adventitious embryoes, bulblets (small bulbs) from the tissue pieces or cultured cells of plants. Referring more in this regard, it is possible to mulitplicate the seedlings of plant in a great amount by rooting adventitious buds or flakes of bulblets into plant bodies, cutting them into tissue pieces, and applying to the tissue culture the culturing method as described above. Further, when usual cultivation is applied to the plants obtained by the method of the present invention, it is possible to grow intact, stable and sound plant bodies.

The present invention will now be described with reference to the reference examples.

### Reference Examples 1 - 10

After sterilizing slices of tomato hypocotyl, slices of egg plant hypocotyl, slices of carrot hypocotyl, slices of onion scaly leaves, slices of soybean leaves, slices of radish hypocotyl, slices of cabbage hypocotyl, slices of basil leaves, slices of bluebottle and slices of rudobekia stalks in an aqueous solution of 70 % ethanol and sodium hyperchlorite (effective chlorine amount 1 %) and cutting them to about 1 cm diameter or about 1 cm length, aseptic Murashige - Skoog agar medium (1962) (agar concentration 0.8 %) at pH 5.6 containing 3 % sucrose, 10⁻⁷M naphthalene acetic acid, 10⁻⁶M benzyl adenine was prepared. Then, the slices as described above were added to the medium and, when the culturing products were left Just after the start of the culture in an aseptic nitrogen gas atmosphere at a room temperature for 30 minutes (aerophobic treatment) and then cultured at 25°C in a bright place for three weeks, the results as shown in Table 1 were obtained for the number of adventitious buds and the number of plants formed per slice.

### Comparative Examples 20 - 29

The tissue culture was conducted in the same procedures as in Reference Examples 1 - 10 except for not conducting aerophobic treatment as in Referebce Examples 1 - 10 and the result are shown in Table 1.

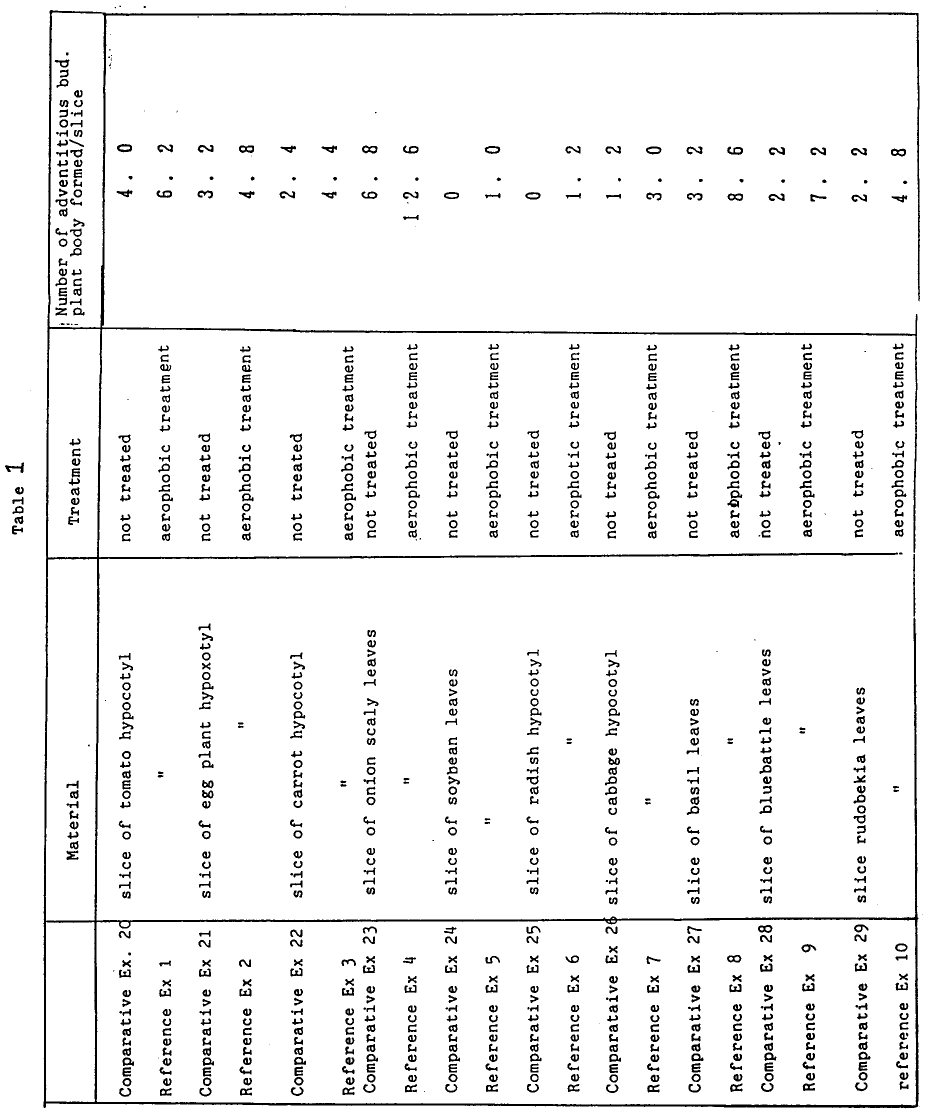

### Reference Examples 11 - 15

The same procedures as in Reference Example 1 were conducted except for using slices of potato leaves, slice of flax hypocotyl, slice of tulip scale, callus cells derived from rice immature embryoes and slices of cucumber leaves as the material in Reference Examples 1.

### Comparative Examples 30 - 34

The sample procedures as in Reference Examples 11 - 15 were conducted excepting for not applying aerophobic treatment in Reference Examples 11 - 15 and the results are shown in Table 2.

**Table 2**

| | Material | Treatment | Number of adventitious bud, plant body formed/slice |
|---|---|---|---|
| Comparative Example 30 | slice of potato leaves | not-treated | 2.0 |
| Reference Example 11 | " | aerophobic treatment | 5.4 |
| Comparative Example 31 | slice of flax hypocotyl | not-treated | 6.2 |
| Reference Example 12 | " | aerophobic treatment | 12.8 |
| Comparative Example 32 | slice of tulip scale | not-treated | 4.2 |
| Reference Example 13 | " | aerophobic treatment | 7.6 |
| Comparative Example 33 | rice callus cells | not-treated | 0.6 |
| Reference Example 14 | " | aerophobic treatment | 2.2 |
| Comparative Example 34 | slice of cucumber leaves | not-treated | 2.4 |
| Reference Example 15 | " | aerophobic treatment | 3.8 |

## Claims

1. A method of multiplicating seedlings of a plant which comprises treating tissue pieces or cultured cells of a plant belonging to Papaveraceae, Solanaceae except for Torenia, Umbelliferae, Rosaceae, Liliaceae except for Lilium, Compositae, Geraniaceae, Cucurbitaceae or Gramineae in a gas atmosphere containing 0 to less than 5% oxygen at a temperature from 15 to 30°C for 30 to 90 minutes and tissue culturing the treated tissue pieces or cultured cells in a medium not containing at least one calcium ionophore or cyclic AMP.

2. A method according to claim 1 wherein the gas atmosphere comprises carbon dioxide, nitrogen and/or argon.

3. A method according to claim 1 or 2 wherein the plant is egg plant, potato, carrot, cabbage, onion, soybean, radish, basil, bluebottle, rudbeckia, tulip, flax, cucumber or rice.

## Patentansprüche

1. Verfahren zur Vermehrung von Pflanzensämlingen, bei dem Gewebestücke oder kultivierte Zellen einer Pflanze, die zu den Papaveraceae, den Solanaceae außer Torenia, den Umbelliferae, den Rosaceae, den Liliaceae außer Lilium, den Compositae, den Geraniaceae, den Cucurbitaceae oder den Gramineae gehört, für 30 bis 90 Minuten bei einer Temperatur von 15 bis 30°C behandelt werden in einer Gasatmosphäre, die weniger als 5 % Sauerstoff enthält, und die behandelten Gewebestücke oder kultivierten Zellen in einem Medium, das zumindestens kein Calciumionophor oder cyclisches AMP enthält, in Gewebekultur kultiviert werden.

2. Verfahren nach Anspruch 1, wobei die Gasatmosphäre Kohlendioxid, Stickstoff und/oder Argon enthält.

3. Verfahren nach Anspruch 1 oder 2, wobei die Pflanze Aubergine, Kartoffel, Karotte, Kohl, Zwiebel, Sojabohne, Rettich, Basilikum, Kornblume, Sonnenhut, Tulpe, Flachs, Gurke oder Reis ist.

## Revendications

1. Procédé de multiplication de plants d'un végétal, qui comporte le fait de traiter des fragments de tissus, ou des cellules cultivées, provenant d'un végétal appartenant aux papavéracées, solanacées (à l'exception du genre Torenia), ombellifères, rosacées, liliacées (à l'exception du genre Lilium), composacées, géraniacées, cucurbitacées ou graminacées, dans une atmosphère de gaz contenant de 0 % à moins de 5 % d'oxygène, à une température de 15°C à 30°C et pendant 30 à 90 minutes, et le fait de cultiver les fragments de tissus ainsi traités, ou les cellules cultivées ainsi traitées, dans un milieu qui ne contient pas en même temps un ionophore des ions de calcium et de l'AMP cyclique.

2. Procédé conforme à la revendication 1, dans lequel l'atmosphère de gaz comporte du dioxyde de carbone, de l'azote et/ou de l'argon.

3. Procédé conforme à la revendication 1 ou 2, dans lequel le plant de végétal est un plant d'aubergine, de pomme de terre, de carotte, de chou, d'oignon, de soja, de radis, de basilic, de bleuet, de rudbeckia, de tulipe, de lin, de concombre ou de riz.
